# EUROPÄISCHE PATENTANMELDUNG

(11) **EP 4 378 391 A1**
(43) Veröffentlichungstag der Anmeldung: **05.06.2024**
(21) Anmeldenummer: 23159901.0
(22) Anmeldetag: 03.03.2023
(51) Int. Cl.: A61B 5/18, A61B 5/00

(54) **SITZEINRICHTUNG UND MODULARES SYSTEM MIT EINER DERARTIGEN SITZEINRICHTUNG**

(30) Priorität: 30.11.2022 DE 102022131707
(71) Anmelder: Hamberger Industriewerke GmbH, 83071 Stephanskirchen (DE)
(72) Erfinder:
(74) Vertreter: Winter, Brandl - Partnerschaft mbB

(57) **Zusammenfassung**

Offenbart sind eine Sitzeinrichtung und ein modulares System mit zumindest einer derartigen Sitzeinrichtung, bei der in eine Sitzauflage, beispielsweise eine Sitzfläche 2, eine Armlehne 6, 8, eine Rückenlehne 10 und/oder eine Fußstütze 18, eine Sensorik 12, 14, 16, 20 zur Erfassung von Vitalparametern integriert ist.

## Beschreibung

Die Erfindung betrifft eine Sitzeinrichtung und ein modulares System, das mit zumindest einer derartigen Sitzeinrichtung ausgeführt ist.

Seit einigen Jahren besteht ein Trend dahingehend, WC-Sitzgarnituren, mit zusätzlichen Funktionen, wie beispielsweise einer Sitzheizung auszuführen. In dem Patent DE 10 2016 108 379 B4 der Anmelderin ist eine WC-Sitzgarnitur beschrieben, bei der in einem WC-Sitz eine Sensorik zur Erfassung physiologischer oder gesundheitsspezifischer Parameter/Kenngrößen einer das WC nutzenden Person erfasst werden.

Derartige Einrichtungen haben den Nachteil, dass für die Erfassung bestimmter gesundheitsspezifischer Parameter (Vitalparameter) mehr Zeit erforderlich ist, als bei der üblichen Nutzung eines WCs verstreicht, so dass die Erfassung dieses Vitalparameters nicht mit der erforderlichen Präzision erfolgt. Des Weiteren ist es auch schwierig, die Person zu motivieren, zur Kontrolle oder Erfassung derartiger Vitalparameter ein WC zu benutzen.

Demgegenüber liegt der Erfindung die Aufgabe zugrunde, eine Einrichtung zu schaffen, mit der die Erfassung von Vitalparametern im Alltag komfortabel erfolgen kann.

Diese Aufgabe wird durch eine Sitzeinrichtung mit den Merkmalen des Patentanspruches 1 und ein modulares System mit zumindest einer derartigen Sitzeinrichtung gelöst.

Vorteilhafte Weiterbildungen der Erfindung sind Gegenstand der Unteransprüche.

Die erfindungsgemäße Sitzeinrichtung hat eine Sitzauflage, in die eine Sensorik zur Erfassung von Vitalparametern einer die Sitzauflage nutzenden Person integriert ist. Es handelt sich dabei um eine herkömmliche Sitzeinrichtung, beispielsweise um einen Stuhl, der in den Wohnbereich integriert ist und von der Person im täglichen Leben und für längere Zeit genutzt wird, so dass für die Erfassung der Vitalparameter genügend Zeit in einer komfortablen Umgebung zur Verfügung steht.

Unter dem Begriff "Sitzauflage" wird dabei jede Einrichtung eines Sitzes oder dergleichen verstanden, auf der ein Körperteil der nutzenden Person abgestützt ist. Der Begriff "Sitzauflage" ist somit nicht auf eine Sitzfläche im herkömmlichen Sinn beschränkt. Dementsprechend kann eine derartige Sitzauflage ein Sitzpolster, eine Armlehne, eine Fußstütze und/oder eine Rückenlehne sein.

Dabei kann diese Sitzauflage ein die Sensorik tragendes Polster, eine Einlage oder eine Matte sein, die Teil der Sitzauflage ist oder diese ausbildet.

Die Anwendung der Sitzeinrichtung ist besonders flexibel, wenn die Sitzauflage auswechselbar ist, so dass beispielsweise durch Wechseln der Sitzauflage unterschiedliche Sensoren in Wirkeingriff gebracht werden können.

Die mit den Sensoren ausgebildete Sensorik kann ausgelegt sein, ein EKG, den Blutdruck, das Herzschlagvolumen, den SpO2-Wert, das Gewicht, die Körpertemperatur, eine Stoffwechselerkrankung, bspw. Diabetes oder dergleichen zu erfassen. Dabei wird die Messwerterfassung durch die Positionierung der Sensorik in dem jeweils optimalen Bereich, beispielsweise in dem Bereich des Sitzpolsters, der Armlehne, der Rückenlehne oder aber auch im Bereich einer Fußstütze optimiert, so dass beispielsweise die Erfassung des EKGs im Kopfbereich, Armbereich, Oberschenkelbereich, Fußbereich ermöglicht ist, um mit Hilfe des EKGs beispielsweise Durchblutungsstörungen zu beurteilen.

Wie vorstehend erwähnt, ist eine variable Vitalparametererfassung durch Bereitstellen auswechselbarer Sitzauflagen mit unterschiedlichen Sensoren möglich.

Bei einem Ausführungsbeispiel der Erfindung ist die Datenauswertung weiter vereinfacht, wenn eine Datenübertragungseinrichtung in die Sitzeinrichtung integriert ist. Diese Datenübertragungseinrichtung ist derart ausgelegt, dass die mittels der Sensorik erfassten Vitalparameter, vorzugsweise kabellos, beispielsweise per Funk oder WLAN an ein Handheld oder eine Auswerteeinheit oder einen Zentralrechner zur Datenauswertung oder Visualisierung der Ergebnisse übertragen werden.

Dabei ist es besonders bevorzugt, wenn diese Datenübertragungseinrichtung, beispielsweise ein Funkmodul, in die Sitzauflage integriert ist.

Die Nutzung der Sitzeinrichtung ist besonders flexibel, wenn diese mit einer Einrichtung zur Identifikation der nutzenden Person ausgeführt ist, so dass die Sitzeinrichtung von mehreren Personen nutzbar ist.

Die Sitzeinrichtung ist bei einem Ausführungsbeispiel mit einer Energieversorgung für die elektrischen Verbraucher, wie die Sensorik und das Funkmodul ausgeführt.

Für derartige Anwendungen bietet sich auch das erfindungsgemäße modulare System an, das mit zumindest einer Sitzeinrichtung gemäß den oben beschriebenen Merkmalen ausgeführt ist, wobei dieser Sitzeinrichtung mehrere Sitzauflagen zugeordnet sind, in die unterschiedliche Sensoren integriert sind und die wahlweise an die Sitzeinrichtung ansetzbar sind. Dementsprechend kann durch geeignete Positionierung ausgewählter Sitzauflagen im Sitzpolster, der Armlehne, der Rückenlehne oder der Fußstütze eine sehr variable Vitalparametererfassung für unterschiedliche Personen, aber auch für unterschiedliche Zielrichtungen erfolgen.

Im Folgenden werden bevorzugte Ausführungsbeispiele der Erfindung anhand einer schematischen Zeichnung erläutert. Diese zeigt eine als Stuhl ausgeführte erfindungsgemäße Sitzeinrichtung.

Bei dem im Folgenden beschriebenen Ausführungsbeispiel ist die erfindungsgemäße Sitzeinrichtung als Stuhl 1 ausgeführt. Dieser Stuhl 1 hat einen herkömmlichen Aufbau mit einer Sitzfläche 2, einem Fußgestell 4, zwei Armlehnen 6, 8 und einer Rückenlehne 10. Beim dargestellten Ausführungsbeispiel ist der Stuhl 1 mit einem Rohrgestell ausgeführt, prinzipiell können jedoch auch herkömmliche Bauweisen mit angesetzten Stuhlbeinen und dergleichen verwendet werden. Die Erfindung ist auch keinesfalls auf die Verwendung bei einem Stuhl 1 beschränkt sondern lässt sich überall dort einsetzen, wo eine Person Körperkontakt hat, beispielsweise eine Bank, ein Sessel, ein Hocker, etc.

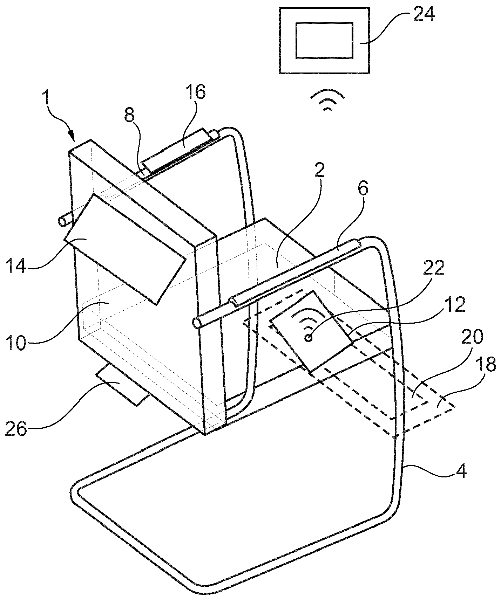

Erfindungsgemäß ist es vorgesehen, entweder in die Sitzfläche 2 oder in die Armlehnen 6, 8 oder in die Rückenlehne 10 eine Sensorik 12, 14 oder 16 zu integrieren, die einen oder mehrere Sensoren zur Erfassung von Vitalparametern, beispielsweise eines EKGs, des Blutdrucks, des SpO2-Wertes, des Herzschlagvolumens, des Körpergewichts, der Körpertemperatur oder des BMI oder einer Stoffwechselerkrankung, bspw. Diabetes aufweist. Die Sensoren sind in geeigneter Weise verschaltet und so positioniert, dass sie in Messkontakt mit der den Stuhl 1 nutzenden Person bringbar sind. In der Figur ist die Positionierung der Sensorik 12, 14, 16 beispielhaft dargestellt. Je nach Zielrichtung kann es hinreichend sein, lediglich eine Sensorik 12, 14, 16 oder eine Kombination derartiger Sensoriken oder eine Vielzahl derartiger Sensorik-Anordnungen vorzusehen. Der Stuhl 1 kann auch, wie in der Figur angedeutet, mit einer Fußstütze 18 ausgeführt sein, auf der eine weitere Sensorik 20 positioniert werden kann, so dass auch eine Vitalparametererfassung über den Fußbereich ermöglicht ist.

Prinzipiell kann auch eine Nackenstütze oder sonstige Komponenten des Stuhls 1 bzw. der Sitzgelegenheit mit einer Sensorik ausgeführt sein.

Für die Befestigung einer derartigen Sensorik 12, 14, 16, 20 stehen eine Vielzahl von Möglichkeiten zur Verfügung. Eine relativ einfache Möglichkeit besteht darin, die Sensorik 20 mit den Sensoren und der zugeordneten Schaltung sowie der Energieversorgung, die beispielsweise induktiv erfolgen kann, in ein Polster oder eine Umhüllung der jeweiligen Sitzkomponente zu integrieren. Vorstellbar ist es auch, die Sensorik als Matte oder Sitzkissen auf den Stuhl 1 aufzulegen, wobei die Signalwertweiterleitung und Energieversorgung dann beispielsweise induktiv über den Stuhl 1 erfolgt.

Erfindungsgemäß wird es bevorzugt, wenn die Sensorik 12, 14, 16 und/oder 20 jeweils auswechselbar am Stuhl 1 gehalten ist, so dass nach Wahl der Sensorik unterschiedliche Vitalparameter erfassbar sind. Die Stromversorgung der Sensoriken 12, 14, 16, 20 kann beispielsweise in herkömmlicher Weise über ein Kabel erfolgen. Dies würde jedoch die Positioniermöglichkeiten des Stuhls 1 einschränken, da dieser dann in der Nähe einer Steckdose angeordnet sein muss. Dementsprechend wird es bevorzugt, die Stromversorgung zur Sitzeinrichtung hin kabellos, bspw. induktiv durchzuführen. Beispiele hierfür sind beispielsweise in der Patentanmeldung EP 3 960 054 A1 der Anmelderin beschrieben.

Selbstverständlich ist auch eine in der Figur angedeutete Energieversorgung 26 über Akkumulatoren oder sonstige Energiequellen, wie beispielsweise Solarpanel oder dergleichen vorstellbar.

Beim dargestellten Ausführungsbeispiel wird vorzugsweise die Sensorik 12, 14, 16, 20 mit einer integrierten Auswerteeinheit ausgeführt. Die von dieser Auswerteeinheit erfassten Daten können dann über ein Funkmodul 22 an ein Tablet 24 oder eine zentrale Recheneinheit übertragen werden, so dass der Nutzer dann nach Auswertung der Daten über ein Display Informationen über die Vitalparameter oder seinen Gesundheitszustand erhält.

Wie eingangs erläutert, kann der Stuhl 1 auch mit einer Identifikationseinrichtung ausgeführt sein, über die erfasst werden kann, welche Person den Stuhl 1 nutzt, so dass die erfassten Vitalparameter entsprechend auch einem dieser Person zugeordneten Speichermedium zugeordnet sind, so dass auch eine individuelle langfristige Datenerfassung ermöglicht ist. Die Identifikation kann auch durch die Person durch Eingeben einer ID in das Tablet oder in eine Eingabeschnittstelle des Stuhls 1 erfolgen. Eine unerwünschte Messung kann bspw. dadurch verhindert werden, dass diese nur nach erfolgter Identifikation/Eingabe/Aktivierung durchgeführt wird.

Eine weitere interessante Anwendungsmöglichkeit besteht darin, das erfindungsgemäße Konzept in Fahrzeugsitzen zu realisieren, so dass entsprechend das Fahrzeug nutzende Personen im Hinblick auf ihre Vitalparameter und Vitalfunktionen überwacht werden können. So könnte die Sensorik beispielsweise auch derart ausgelegt sein, dass aus ungewöhnlichen Messwerten des Fahrers auf eine Müdigkeit oder sonstige, die Fahrsicherheit beeinträchtigende Umstände geschlossen werden kann, so dass entsprechend eine Warnung an den Fahrer abgegeben wird.

Offenbart sind eine Sitzeinrichtung und ein modulares System mit zumindest einer derartigen Sitzeinrichtung, bei der in eine Sitzauflage, beispielsweise eine Sitzfläche, eine Armlehne, eine Rückenlehne und/oder eine Fußstütze, eine Sensorik zur Erfassung von Vitalparametern integriert ist.

### Bezugszeichenliste:

- 1: Stuhl
- 2: Sitzfläche
- 4: Sitzgestell
- 6: Armlehne
- 8: Armlehne
- 10: Rückenlehne
- 12: Sensorik
- 14: Sensorik
- 16: Sensorik
- 18: Fußstütze
- 20: Sensorik
- 22: Funkmodul
- 24: Tablet
- 26: Energieversorgung

## Patentansprüche

1. Sitzeinrichtung mit einer Sitzauflage und mit einer Sensorik (12, 14, 16, 20) zur Erfassung von Vitalparametern einer die Sitzauflage nutzenden Person, wobei die Sensorik (12, 14, 16, 20) in die Sitzauflage integriert ist.

2. Sitzeinrichtung nach Patentanspruch 1, wobei die Sitzauflage ein(e) Sitzpolster/Sitzfläche (2), eine Armlehne (6, 8), eine Rückenlehne (10) und/oder eine Fußstütze (18) ist.

3. Sitzeinrichtung nach Patentanspruch 2, wobei die Sitzauflage ein die Sensorik (12, 14, 16, 20) tragendes oder aufnehmendes Polster oder eine die Sensorik (12, 14, 16, 20) tragende oder aufnehmende Einlage oder Matte hat.

4. Sitzeinrichtung nach einem der vorhergehenden Patentansprüche, wobei die Sitzauflage auswechselbar ist.

5. Sitzeinrichtung nach einem der vorhergehenden Patentansprüche, wobei die Sensorik (12, 14, 16, 20) ausgelegt ist, ein EKG, den Blutdruck, das Herzschlagvolumen, den SpO2-Wert, den BMI-Index, das Gewicht, die Körpertemperatur oder dergleichen oder eine Stoffwechselerkrankung, bspw. Diabetes zu erfassen.

6. Sitzeinrichtung nach Patentanspruch 5, wobei Sitzauflagen mit unterschiedlichen Sensoren bereitgestellt sind.

7. Sitzeinrichtung nach einem der vorhergehenden Patentansprüche, mit einer Energieversorgung (26) und/oder mit einer Datenübertragungseinrichtung, die ausgelegt ist, die mittels der Sensorik (12, 14, 16, 20) erfassten Daten, vorzugsweise kabellos, beispielsweise per Funk, WLAN an ein Handheld oder eine Auswerteeinheit oder einen Zentralrechner zu übertragen oder die ermittelten Parameter auf einem Display anzuzeigen.

8. Sitzeinrichtung nach Patentanspruch 7, wobei die Energieversorgung (26) und/oder die Datenübertragungseinrichtung in die Sitzauflage integriert ist.

9. Sitzeinrichtung nach einem der vorhergehenden Patentansprüche, mit einer Einrichtung zur Identifikation der nutzenden Person.

10. Modulares System mit zumindest einer Sitzeinrichtung nach einem der vorhergehenden Patentansprüche und mehreren Sitzauflagen, in die unterschiedliche Sensoren (12, 14, 16, 20) integriert sind und die wahlweise an die Sitzeinrichtung ansetzbar sind.
